# DEMANDE DE BREVET EUROPEEN

(11) **EP 2 093 983 A1**
(43) Date de publication de la demande: **26.08.2009**
(21) Numéro de dépôt: 09153567.4
(22) Date de dépôt: 25.02.2009
(51) Int. Cl.: H04M 1/725, G06Q 30/00

(54) **Procédé de prise de controle de l'affichage sur un écran par un terminal radio**

(30) Priorité: 25.02.2008 FR 0851173
(71) Demandeur: Alcatel Lucent, 75008 Paris (FR)
(72) Inventeur: Rouffet, Denis, 78141, VELIZY (FR)
(74) Mandataire: Hedarchet, Stéphane

(57) **Abrégé**

Un procédé est dédié à l'ouverture d'une session de communication entre un terminal radio (MS), connecté à un réseau radio (R1), et un terminal de communication fixe (TC), couplé à au moins un écran cible (EC) et connecté à un réseau de communication (R2). Ce procédé consiste à envoyer avec le terminal radio (MS) un identifiant affiché sur l'écran cible (EC) pour ouvrir une session de communication entre ce terminal radio (MS) et le terminal de communication fixe (TC), puis à contrôler l'affichage d'informations sur l'écran cible (EC) au moyen du terminal radio (MS).

## Description

L'invention concerne le domaine de la fourniture d'informations à des personnes.

Il arrive fréquemment qu'une personne recherche des informations lorsqu'elle se déplace, par exemple dans une ville, un bâtiment, un véhicule de transport en commun ou un lieu public, ou bien lorsqu'elle regarde un programme de télévision. Pour ce faire, plusieurs solutions existent déjà.

Ainsi, une personne peut par exemple se servir de son téléphone mobile (ou cellulaire - ou d'un autre type de terminal de communication radio communicant) pour appeler un service de renseignements téléphonique ou accéder à un site Internet d'informations.

Une autre solution consiste à mettre à la disposition des personnes des informations stockées dans des ordinateurs (ou bornes informatiques) couplé(e)s à des écrans interactifs munis d'une (ou combinés à une) interface homme/machine pour la sélection et la navigation (comme par exemple des touches de commande (éventuellement tactiles (sur l'écran)) et/ou une souris).

Encore une autre solution consiste à se placer à proximité d'une borne informatique radio (par exemple de type bluetooth ou Wi-Fi) pour télécharger dans un téléphone mobile (ou tout autre type de terminal de communication radio communicant) adapté à cet effet des informations stockées dans cette borne.

Encore une autre solution consiste à photographier un cryptogramme situé sur une affiche ou un panneau au moyen de l'appareil photographique qui est intégré dans un téléphone mobile (ou tout autre type de terminal de communication radio communicant), de manière à alimenter une application de services interne capable d'établir une communication avec un serveur de services afin de lui communiquer le cryptogramme photographié pour qu'il transmette au téléphone mobile les informations qui lui correspondent.

Encore une autre solution consiste à utiliser des panneaux sur lesquels des informations défilent régulièrement. Mais, il n'est pas possible d'interagir avec ces panneaux et donc la personne qui lit le panneau est contrainte d'attendre que l'information qui l'intéresse défile et elle n'a aucun moyen d'arrêter le défilement ni d'en inverser le sens pour revenir en arrière.

Encore une autre solution consiste à mettre en oeuvre pour un téléphone mobile (ou tout autre type de terminal de communication radio communicant) une application de service reposant sur la localisation (par exemple de type LBS (pour « Location Based Service »)), afin de localiser ce téléphone mobile et de lui transmettre des informations relatives au lieu dans lequel il est situé. Cette solution est notamment utilisée dans les applications qui permettent le téléchargement de points intéressants (ou POls (pour « Points of Interest » en anglais)) afin de les afficher sur une carte.

Chaque solution précités présente au moins un inconvénient qui la rend difficilement utilisable par le plus grand nombre et/ou pas assez conviviale et/ou pas assez informative.

L'invention a pour but de proposer une solution alternative aux solutions connues.

Elle propose à cet effet un procédé dédié à l'ouverture d'une session de communication entre un terminal (de communication) radio, connecté à un réseau (de communication) radio, et un terminal de communication fixe, couplé à au moins un écran cible et connecté à un réseau de communication (qui est éventuellement le même que le réseau radio).

Ce procédé se caractérise par le fait qu'il consiste :
- à envoyer avec le terminal radio un identifiant (éventuellement de communication) qui est affiché sur l'écran cible afin d'ouvrir une session de communication entre ce terminal radio et le terminal de communication fixe, puis
- à contrôler l'affichage d'informations sur l'écran cible à l'aide du terminal radio.

Le procédé selon l'invention peut comporter d'autres caractéristiques qui peuvent être prises séparément ou en combinaison, et notamment :
- on peut transférer le contrôle de l'affichage d'informations au terminal radio;
   ➢ on peut envoyer à un serveur, avec le terminal radio, un identifiant qui est choisi dans une liste affichée sur l'écran cible, puis on peut ouvrir la session de communication au moyen de ce serveur, et on peut procéder au transfert du contrôle d'affichage au moyen de ce serveur ;
- on peut composer l'identifiant avec le terminal radio lorsqu'il constitue un identifiant de communication ;
- on peut utiliser des moyens du terminal radio (constituant une interface homme/machine), pour effectuer des opérations permettant d'agir sur certaines au moins des informations affichées sur l'écran cible et/ou de provoquer l'affichage sur l'écran cible de certaines informations stockées ;
- en cas d'affichage sur l'écran cible d'informations accompagnées d'un autre identifiant donnant accès à d'autres informations stockées, on peut provoquer l'affichage de ces autres informations stockées en envoyant (éventuellement en composant) cet autre identifiant avec le terminal radio ;
- on peut afficher sur un écran d'affichage du terminal radio certaines au moins des informations qui sont affichées sur l'écran cible ;
- on peut déclencher au moins une application de télécommunications, comme par exemple une application de mise en communication ou une application permettant de stocker certaines au moins des informations qui sont affichées sur l'écran cible dans des moyens de stockage désignés par l'usager du terminal radio ;
- on peut transmettre au terminal radio des messages audio associés aux informations affichées afin qu'ils, puissent être écoutés par son usager (et éventuellement stocker ces messages audio dans le terminal radio) ;
- on peut mettre fin à la session de communication en cas de détection d'une absence d'opération de contrôle d'affichage avec le terminal radio pendant une durée supérieure à un seuil choisi ;
- on peut mettre fin à la session de communication lorsque l'usager du terminal radio interrompt la communication :

- on peut facturer à l'usager du terminal radio la prise de contrôle de l'affichage ;
- on peut stocker dans le terminal de communication fixe certaines au moins des informations qui sont destinées à être affichées sur l'écran cible ;
- on peut stocker dans le serveur certaines au moins des informations qui sont destinées à être affichées sur l'écran cible.

L'invention propose également un serveur de réseau de communication chargé, en cas d'établissement d'une communication avec un terminal (de communication) radio connecté à un réseau (de communication) radio, consécutivement à l'envoi par ce terminal radio d'un identifiant affiché sur un écran cible couplé à un terminal de communication fixe connecté à un réseau de communication, d'ouvrir une session de communication entre le terminal radio et le terminal de communication fixe, puis de transférer à ce terminal radio le contrôle de l'affichage d'informations sur l'écran cible.

Le serveur selon l'invention peut comporter d'autres caractéristiques qui peuvent être prises séparément ou en combinaison, et notamment :
- il peut être chargé, en cas d'envoi (éventuellement de composition) par le terminal radio pendant la session de communication d'un autre identifiant affiché sur l'écran cible et donnant accès à d'autres informations stockées, d'autoriser l'accès à ces autres informations stockées de sorte qu'elles puissent être affichées sur l'écran cible ;
- il peut être chargé d'autoriser la transmission au terminal radio de certaines au moins des informations qui sont affichées sur l'écran cible, en vue de leur affichage sur un écran d'affichage de ce terminal radio ;
- il peut être chargé de déclencher au moins une application de télécommunications, comme par exemple une application de mise en communication de l'usager du terminal radio avec un autre usager ou un équipement de communication (comme par exemple un répondeur ou un serveur de contenus), ou bien une application permettant de stocker certaines au moins des informations qui sont affichées sur l'écran cible dans des moyens de stockage désignés par l'usager du terminal radio ;
- il peut être chargé de transmettre au terminal radio des messages audio associés aux informations affichées afin qu'ils puissent être écoutés par son usager (et éventuellement stockés localement) ;
- il peut être chargé de mettre fin à la session de communication en cas de détection d'une absence d'opération de contrôle d'affichage avec le terminal radio pendant une durée supérieure à un seuil choisi ;
- il peut être chargé de mettre fin à la session de communication lorsque l'usager du terminal radio interrompt la communication ;
- il peut être chargé de contrôler la facturation à l'usager du terminal radio de la prise de contrôle de l'affichage ;
- il peut comprendre des moyens de stockage stockant certaines au moins des informations qui sont destinées à être affichées sur l'écran cible ;
- il peut être chargé d'autoriser la transmission à une adresse désignée par l'usager du terminal radio de certaines au moins des informations qui sont affichées sur l'écran cible, en vue de leur stockage dans des moyens de stockage.

D'autres caractéristiques et avantages de l'invention apparaîtront à l'examen de la description détaillée ci-aprés, et des dessins annexés, sur lesquels:
- la figure 1 illustre de façon très schématique une vitrine de magasin derrière laquelle se trouve installe un écran cible connecté à un terminal de communication filaire connecté à un réseau filaire, et devant laquelle est momentanément située une personne équipée d'un téléphone mobile propre à se connecter à un réseau mobile auquel est connecté un serveur selon l'invention,
- la figure 2 illustre de façon très schématique un exemple d'informations générales affichées sur un écran cible,
- la figure 3 illustre de façon très schématique un exemple de groupe de huit fenêtres d'informations, d'icônes, symbolisant des touches à actionner pour accéder aux différentes fenêtres d'informations, et de second identifiant de communication, permettant d'accéder à d'autres informations, qui s'affichent sur un écran cible à la prise de contrôle de son affichage, et
- la figure 4 illustre de façon très schématique un exemple de groupe de trois fenêtres d'informations qui s'affichent sur un écran cible consécutivement à la composition du second identifiant de communication.

Les dessins annexés pourront non seulement servir à compléter l'invention, mais aussi contribuer à sa définition, le cas échéant.

L'invention a notamment pour objet de permettre la mise à disposition d'informations sur un écran dit cible, couplé à un (ou faisant partie d'un) terminal de communication fixe connecté à un réseau de communication (filaire ou non filaire), pour une personne placée devant cet écran cible et munie d'un terminal de communication radio (mobile ou portable) connecté à un réseau de communication radio (non filaire).

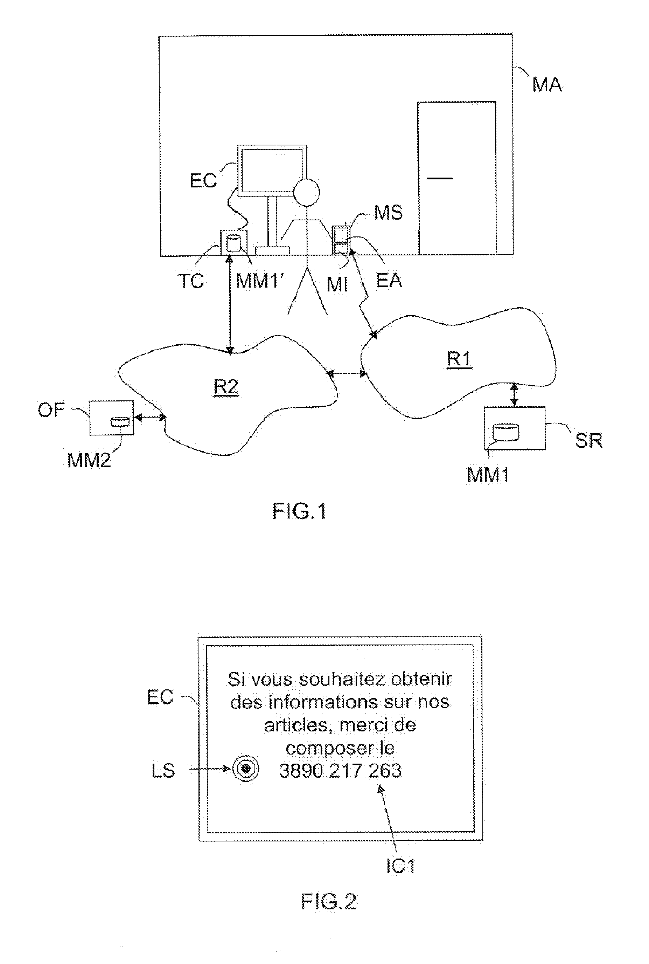

On se réfère tout d'abord à la figure 1 pour présenter l'invention dans le cadre d'un exemple d'application non limitatif,

Dans l'exemple illustré on a représenté un écran cible EC connecté à un terminal de communication fixe TC, lui même connecté à un réseau de communication R2 (filaire ou non filaire), et tous les deux installés dans une vitrine d'un magasin MA devant laquelle est momentanément située une personne équipée d'un terminal de communication radio (mobile ou portable) MS propre à se connecter à un réseau de communication radio (non filaire) R1 connecté (directement ou indirectement) au réseau de communication R2.

Dans ce qui suit, on considère à titre d'exemple non limitatif que le réseau de communication R2 auquel est connecté le terminal de communication fixe TC est distinct du réseau de communication radio auquel est connecté le terminal de communication radio (mobile ou portable) MS. Mais, cela n'est pas obligatoire. En effet, il peut s'agir d'un même réseau de communication radio.

Par ailleurs, on considère dans ce qui suit, à titre d'exemple non limitatif, que le réseau de communication R2 auquel est connecté le terminal de communication fixe TC est un réseau filaire, par exemple de type ADSL (offrant éventuellement un accès lP). Mais, un terminal de communication fixe TC peut être connecté à d'autres types de réseau de communication, et notamment un réseau filaire, tel qu'un réseau à câbles ou à fibres optiques ou un réseau non filaire, tel qu'un réseau mobile (ou cellulaire), un réseau local (standards WLAN (y compris le WiFi) et WiMAX)), un réseau satellitaire; (par exemple conforme à un standard DVB ou analogue) ou un réseau hybride. Il peut être également connecté à plusieurs moyens de communication simultanément, lui permettant ainsi de recevoir par exemple, d'une part, des informations communes par exemple grâce à un réseau satellitaire de diffusion (« broadcast »), et d'autre part, les informations dédiées à son utilisateur après sa prise de contrôle de l'affichage par exemple grâce à un réseau ADSL.

En outre, on considère dans ce qui suit, à titre d'exemple non limitatif, que le réseau de communication radio R1 auquel est connecté le terminal de communication radio MS est un réseau non filaire, par exemple de type mobile (ou cellulaire), offrant éventuellement un accès IP. D'une manière générale, le terminal de communication radio MS peut être connecté à n'importe quel type de réseau radio, et notamment à un réseau mobile (ou cellulaire (GSM, CDMA, WCDMA, 3G LTE, ou leurs évolutions) ou à un réseau local (standards WLAN (y compris le WiFi) et WiMAX)) ou encore à un réseau satellitaire.

De plus, on considère dans ce qui suit, à titre d'exemple non limitatif, que le terminal de communication fixe TC est un modem connecté à au moins un écran dit cible EC, et que le terminal de communication radio (mobile ou portable) MS est un téléphone mobile (ou cellulaire) disposant d'un écran d'affichage EA et d'une interface homme/machine MI pour la saisie de commandes d'opérations ou d'actions. Mais, l'invention n'est pas limitée à ces types de terminaux de communication. En effet, le terminal de communication fixe TC pourra également être un ordinateur fixe (ou éventuellement portable), un téléviseur (ou écran) numérique équipé d'un (ou comprenant un) modem, ou un récepteur de contenus équipé d'un module de communication filaire ou non filaire et éventuellement associé à un projecteur, et le terminal de communication radio (mobile ou portable) MS pourra également être un assistant numérique personnel (ou PDA, y compris un « pocket PC ») ou un ordinateur portable (bien que cela ne soit pas très pratique) ou n'importe quelle évolution des types précités.

On notera notamment que le terminal de communication radio MS peut être également un équipement muni d'un récepteur GPS situé dans un véhicule (amovible ou non) et permettant couverture de sessions de communication du fait qu'il comprend un module de communication non filaire intégré ou qu'il peut communiquer avec un module de communication externe de type portable par exemple par une liaison Bluetooth. Cela permet d'utiliser l'invention à bord d'un véhicule qui est arrêté devant un écran cible EC, et par exemple de mettre en oeuvre une application d'indication et de réservation de places de parking avant de pénétrer dans celui-ci et de communiquer à l'utilisateur qui a pris le contrôle de l'écran cible EC la localisation de la place réservée.

On notera que le terminal de communication fixe TC pourrait être de type radio (non filaire).

L'invention propose un procédé permettant d'ouvrir une session de communication entre un terminal de communication radio MS (ici le téléphone mobile d'une personne) et un terminal de communication fixe TC (ici un modem), couplé à au moins (ou muni d'au moins) un écran cible EC.

Dans ce qui suit, on considère à titre d'exemple non limitatif que le modem TC est couplé à un seul écran cible EC. Mais, il pourrait être couplé à plusieurs (au moins deux) écrans cibles.

Le procédé selon l'invention est mis en oeuvre lorsqu'une personne munie d'un téléphone mobile TC se trouve momentanément située devant un écran cible EC sur lequel se trouvent affichées des informations générales contenant au moins un identifiant (éventuellement de communication) IC1, comme par exemple un numéro de téléphone (ou bien une adresse Internet, ou analogue) ou un logo ou encore un cryptogramme, permettant d'accéder à des informations, et que cette personne souhaite accéder à certaines au moins desdites informations.

On a schématiquement représenté sur la figure 2, un exemple non limitatif d'informations générales pouvant être affichées sur un écran cible EC. Dans cet exemple, le message affiché invite les personnes qui le lisent à composer le numéro 3890 217263 afin d'obtenir des informations sur les articles qui sont vendus dans le magasin MA. On notera que dans cet exemple l'identifiant [C1 est un identifiant de communication qui est accompagné d'un logo (ou symbole) LS qui signale aux personnes l'affiliation à un service. Mais, ce logo LS n'est pas obligatoire. Un tel logo LS peut être notamment utile lorsque l'identifiant de communication n'est pas accompagné d'un message. Dans ce cas, lorsqu'une personne voit l'identifiant de communication et le logo (ou symbole) LS, elle comprend immédiatement que cet identifiant de communication permet d'accéder à des informations qui vont s'afficher sur l'écran cible EC placé devant elle. Le logo ou un cryptogramme peut même servir d'identifiant. Il doit alors être capté par une application photographique du téléphone mobile MS.

Le procédé comprend une première étape au cours de laquelle une personne envoie, avec son téléphone mobile MS, l'identifiant IC1, qui est affiché sur l'écran cible EC devant lequel elle est momentanément située, afin d'ouvrir une session de communication entre son téléphone mobile MS et le modem TC auquel est couplé l'écran cible EC.

On notera que l'identifiant de communication qui est utilisé peut avoir été choisi dans une liste affichée sur l'écran cible EC.

Par ailleurs, on considère dans ce qui suit, à titre d'exemple non limitatif, que l'identifiant IC1 qui est utilisé est un identifiant de communication. Il est donc composé avec l'interface homme/machine MI du téléphone mobile MS. Mais, comme indiqué ci-avant, l'identifiant IC1 peut être un logo ou un cryptogramme qui doit être capté par une application photographique du téléphone mobile MS, puis envoyé.

Le procédé comprend ensuite une seconde étape au cours de laquelle on contrôle l'affichage d'informations sur l'écran cíble EC au moyen du téléphone mobile MS.

De préférence, ce contrôle ce fait par un transfert du contrôle de l'affichage au téléphone mobile MS.

Le contrôle de l'ouverture de la session de communication et du transfert de contrôle d'affichage peut se faire au moyen d'un serveur SR qui est connecté à l'un des réseaux R1 et R2, ou bien accessible à l'un au moins d'entre eux. Dans l'exemple non limitatif illustrè sur la figure 1, le serveur SR est connecté au réseau mobile R1 Mais, il pourrait être connecté au réseau filaire R2,

On notera qu'un tel serveur SR peut éventuellement se présenter sous la forme d'un serveur d'application implanté dans un coeur de réseau, éventuellement de type IMS.

On comprendra qu'un tel serveur SR peut avantageusement contrôler l'ouverture de sessions de communication avec plusieurs terminaux de communication fixes TC, ainsi que le transfert du contrôle de l'affichage de plusieurs écrans cibles EC de chacun de ces terminaux de communication fixes TC. Par conséquent, on peut envisager que plusieurs personnes différentes placées devant des écrans cibles EC différents, couplés à un même terminal de communication fixe TC, puissent prendre en parallèle le contrôle de l'affichage de ces différents écrans cibles EC, si ledit terminal de communication fixe TC le permet. On peut également envisager que plusieurs personnes différentes placées devant un même écran cible EC couplé à un terminal de communication fixe TC puissent prendre en parallèle le contrôle de l'affichage de parties distinctes de cet écran cible EC, si ledit terminal de communication fixe TC et ledit écran cible EC le permettent.

L'identifiant (de communication) IC1 qui est initialement composé (ou envoyé) au moyen du téléphone mobile MS adresse donc non seulement le serveur SR, mais également un terminal de communication fixe TC (ici un modem), ainsi qu'éventuellement un écran cible EC si ce modem TC est couplé à plusieurs écrans cibles EC.

Le serveur SR se charge donc d'ouvrir la session de communication entre le téléphone mobile MS requérant et le modem TC désigné par l'identifiant (de communication) IC1, puis de transférer au téléphone mobile MS le contrôle de l'affichage d'informations sur l'écran cible EC (éventuellement désigné par cet identifiant (de communication) IC1).

Par exemple, consécutivement au transfert du contrôle d'affichage, des premières informations prédéfinies peuvent être automatiquement affichées sur l'écran cible EC objet du transfert.

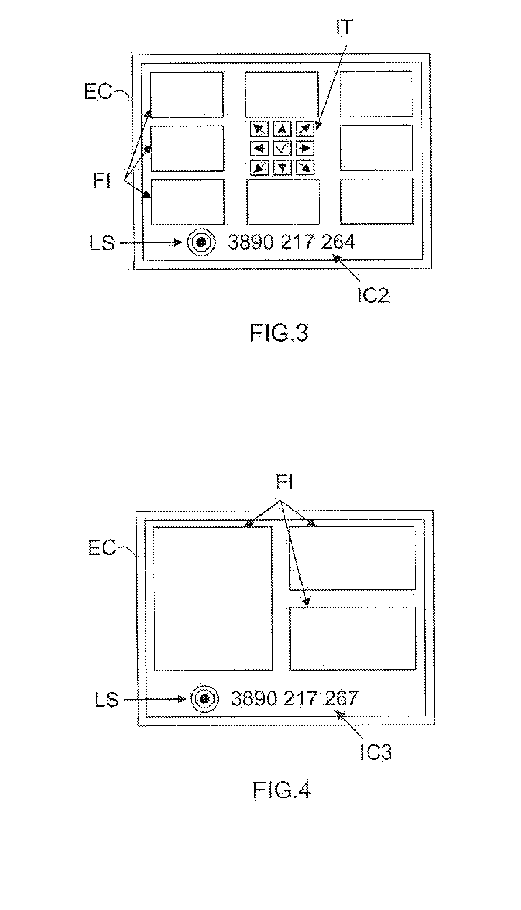

On a schématiquement représenté sur la figure 3, un exemple non limitatif de premières informations affichées sur l'écran cible EG. Dans cet exemple, les premières informations sont réparties dans huit fenêtres d'informations FI et dans des icônes IT qui symbolisent les touches que doit actionner une personne sur son téléphone mobile MS pour accéder aux différentes fenêtres d'informations FI, et sont accompagnées d'un second identifiant (éventuellement de communication) IC2 (3890 217 264) qui permet d'accéder à d'autres informations (éventuellement plus détaillées).

On notera que le second identifiant IC2 peut être également un logo ou un cryptogramme qui doit être capté par une application photographique du téléphone mobile MS, puis envoyé.

Par exemple :
- l'icône ↑ correspond au chiffre 2 du clavier du téléphone mobile MS et permet de sélectionner la fenêtre placée dans la position supérieure centrale.
- l'icône ↖ correspond au chiffre 1 du clavier du téléphone mobile MS et permet de sélectionner la fenêtre placée dans la position supérieure gauche,
- l'icône ↗ correspond au chiffre 3 du clavier du téléphone mobile MS et permet de sélectionner la fenêtre placée dans la position supérieure droite,
- l'icône ← correspond au chiffre 4 du clavier du téléphone mobile MS et permet de sélectionner la fenêtre placée dans la position centrale gauche.
- l'icône → correspond au chiffre 6 du clavier du téléphone mobile MS et permet de sélectionner la fenêtre placée dans la position centrale droite,
- l'icône ↓ correspond au chiffre 8 du clavier du téléphone mobile MS et permet de sélectionner la fenêtre placée dans la position inférieure centrale,
- l'icône ↙ correspond au chiffre 7 du clavier du téléphone mobile MS et permet de sélectionner la fenêtre placée dans la position inférieure gauche,
- l'icône ↘ correspond au chiffre 9 du clavier du téléphone mobile MS et permet de sélectionner la fenêtre placée dans la position inférieure droite, et
- l'icône √ placée au centre correspond au chiffre 5 du clavier du téléphone mobile MS et permet (éventuellement) de valider la sélection d'une fenêtre.

On comprendra que la sélection d'une fenêtre entraîne l'affichage des informations qu'elle contient sur la totalité ou la quasi totalité de l'écran cible EC. Cela est équivalent à une opération de grossissement (ou « zoom »). On notera qu'en sélectionnant une fenêtre cela peut éventuellement provoquer l'affichage additionnel sur l'écran cible EC d'un nouvel identifiant (éventuellement de communication ou logo ou cryptogramme) ICn permettant d'accéder à des informations complémentaires et/ou plus détaillées et/ou à un masque de sélection. Le fait d'actionner une touche du téléphone mobile MS est donc interprété comme une instruction de navigation (ou « browsing ») par le serveur SR et transmis au modem TC puis à la logique de contrôle de l'écran cible EC. Cette logique choisit alors un contenu qui peut être déjà dans la mémoire associée à l'écran cible EC ou qui est envoyé à ce dernier par le serveur SR.

Il est important de noter que les informations qui sont destinées à être affichées sur l'écran cible EC peuvent être stockées dans des moyens de stockage du modem TC (MM1') et/ou du serveur SR (MM1) et/ou d'un autre équipement de réseau (non représenté sur la figure 1).

Les informations du contenu affichées dans les fenêtres d'informations FI sont alors :
- soit celles que cherche la personne et dans ce cas cette dernière les lit et peut demander des services supplémentaires offerts par des applications qui peuvent être déclenchées par le serveur SR (comme par exemple une application de mise en mémoire dans son mobile ou dans un autre équipement de communication, ou une application de mise en communication avec une autre personne ou un équipement de communication, ou encore une application de notification de l'intérêt manifesté par la personne pour un éventuelle suivi par l'entité qui a produit le contenu affiché),
- soit utilisées pour continuer à chercher un contenu plus dètaillé ou différent. Dans ce cas, la personne peut par exemple composer (ou envoyer), avec l'interface homme/machine MI de son téléphone mobile MS. l'identifiant (de communication) IC2, qui est affiché sur l'écran cible EC devant lequel elle est momentanément située. Le fait de composer (ou envoyer) ce nouvel identifiant (de communication) IC2 est interprèté par le serveur SR comme une requête de demande d'accès à d'autres informations que les premières informations qui sont affichées dans les fenêtres d'informations FI. Il s'agit donc également d'une instruction de navigation qui est éventuellement transmise à la logique de contrôle de l'écran cible EC.

On a schématiquement représenté sur la figure 4, un exemple non limitatif de (trois) fenêtres d'informations FI qui sont affichées sur l'écran cible EC consécutivement à la composition (ou l'envoi) d'un nouvel (second) identifiant (éventuellement de communication ou logo ou cryptogramme) IC2 (3890 217 264) affiché sur un écran précédent (ici celui de la figure 3). On notera qu'un nouvel identifiant (éventuellement de communication ou logo ou cryptogramme) IC3 (ici 3890 217 267) peut éventuellement accompagner les nouvelles informations affichées sur l'écran cible EC et permettre d'accéder à encore d'autres informations (éventuellement plus détaillées).

Si une personne désire revenir à des informations précédemment affichées sur l'écran cible EC, elle peut par exemple utiliser la touche de retour en arrière de son téléphone mobile MS qui est couplée à une fonction de mémorisation des dernières actions effectuées et l'on transmet à l'écran cible EC l'ordre d'afficher les informations précédentes. Les méthodes utilisables pour ce retour en arrière sont classiques et bien connues de l'homme de l'art.

On comprendra que dans l'invention le téléphone mobile MS constitue en quelque sorte une télécommande communicante sophistiquée.

Il est important de noter que les informations qui sont affichées sur un écran cible EC ne sont pas forcément statiques (texte ou image). Il peut ègalement s'agir d'images vidéo (éventuellement accompagnées d'une bande son (audio)). Cette dernière peut être transmise au téléphone mobile MS par le serveur SR afin que seul son usager l'entende, évitant ainsi qu'elle soit diffusée dans la rue ou dans un lieu public.

Une session de communication entre un téléphone mobile MS et un modem TC peut se terminer soit à l'initiative de la personne qui utilise le téléphone mobile MS, par actionnement d'une touche de interface homme/machine MI dédiée à l'interruption de communication, soit par détection de l'absence d'opération de contrôle d'affichage issue du téléphone mobile MS pendant une durée qui est supérieure à un seuil choisi (par exemple égal à 30 secondes ou 1 minute). Ce dernier cas nécessite le déclenchement d'une temporisation chaque fois qu'un téléphone mobile MS transmet un signal consécutivement à l'actionnement d'une touche de son interface homme/machine MI. C'est le serveur SR qui peut être chargé de mettre fin à une session de communication lorsqu'il détecte un dépassement du seuil (sil gère la temporisation correspondante) ou l'interruption de la communication avec le téléphone mobile MS.

On notera que l'on peut envisager de transmettre au téléphone mobile MS certaines au moins des informations qui sont affichées sur l'écran cible EC dont il contrôle l'affichage, afin qu'elles soient affichées en parallèle sur son écran d'affichage EA, et/ou des informations complémentaires contenues par exemple dans des messages audio. Cela nécessite bien entendu que la personne qui utilise le téléphone mobile MS actionne au moins une touche de son interface homme/machine MI qui a été désignée par des informations affichées sur l'écran cible EC.

On notera qu'une fois qu'une personne a trouvé les informations qui l'intéressent et qui sont affichées sur l'écran cible EC, un message affiché peut lui proposer de sauvegarder ces informations dans des moyens de stockage MM2 de son choix, par exemple compris dans son téléphone mobile MS ou bien dans un ordinateur fixe (ou mobile) OF qui est connecté à un réseau de communication, comme par exemple le réseau filaire R2 (comme dans l'exemple non limitatif de la figure 1). C'est le serveur SR qui peut être chargé du contrôle du transfert des informations affichées vers les moyens de stockage MM2 désignés.

On notera également que la prise de contrôle d'affichage peut être éventuellement un service payant. Par conséquent, l'invention peut permettre un contrôle de la facturation d'une prise de contrôle d'affichage à l'abonné qui dispose du numéro de téléphone du téléphone mobile MS qui est utilisé. Cette facturation peut porter sur la prise de contrôle et/ou sur la durée de la prise de contrôle et/ou sur la quantité d'informations affichées pendant une session de communication et/ou sur la quantité et/ou la durée de transmission des informations transmises au téléphone mobile MS. Dans certains cas (par exemple celui d'un touriste), il est possible de prendre un abonnement afin d'avoir un accès limité dans le temps (ou non) à un certain nombre d'écrans cibles EC situés à des endroits prédéfinis intéressants. L'interaction avec les écrans cibles EC fournit alors l'accès à des informations vidéo et/ou audio et/ou textuelles dans la langue de l'utitisateur. La facturation peut par exemple être classiquement contrôlée par le serveur SR, ou bien être prise en charge par les mécanismes de facturation au temps ou au volume qui sont classiquement utilisés dans les réseaux mobiles.

Quelques exemples simples et non limitatifs d'application de l'invention sont décrits ci-après.

Un premier exemple correspond à la situation lustrée sur la figure 1. On considère ici que le magasin MA est un magasin de chaussure. La personne qui est devant l'écran cible EC compose (ou envoie) l'identifiant (de communication) IC1 qui est affiché sur cet écran cible EC avec son téléphone mobile MS. Des informations générales s'affichent alors sur l'écran cible EC, et la personne peut naviguer parmi ces informations générales (pour zoomer) ou bien parmi d'autres en composant (ou envoyant) un autre identifiant (de communication) IC2 qui est affiché sur l'écran cible EC avec les informations générales. Par exemple, on peut présenter à la personne tous les modèles de chaussure qui sont en vente dans le magasin MA ou bien on peut proposer à la personne de communiquer sa pointure afin de ne lui présenter que les modèles de chaussure pour lesquels sa pointure est disponible. Il peut alors être proposé à cette personne de sélectionner au moins l'un des modèles de chaussure présentés afin qu'elle puisse les essayer à l'intérieur du magasin MA. La personne ayant alors présélectionné des modèles, ils peuvent alors lui être rapidement présentés dès qu'elle entre dans le magasin MA. Il peut être également envisagé de proposer à cette personne de vérifier si un modèle sélectionné est disponible dans un autre magasin de la même enseigne.

Un deuxième exemple correspond à une situation dans laquelle une personne qui est chez elle regarde l'écran cible EC de son téléviseur qui est connecté à (ou comprend) un modem TC. Par exemple, le programme affiché comporte un identifiant (de communication) permettant d'accéder à des informations qui le décrivent ou qui décrivent des personnes ou objets qui apparaissent. La personne compose (ou envoie) alors cet identifiant (de communication) avec son téléphone mobile MS et des informations générales s'affichent sur l'écran cible EC en remplacement du programme ou bien de façon superposée ou encore en incrustation, et la personne peut éventuellement accéder à d'autres informations (éventuellement plus détaillées) en composant (ou envoyant) un autre identifiant (de communication) affiché, Les informations affichées proviennent sort du réseau de diffusion des programmes de télévision, soit de l'lnternet.

Un troisième exemple correspond à une situation dans laquelle la personne est située devant un écran cible EC implanté dans une rue, un bâtiment, un véhicule de transport en commun ou un lieu public. L'écran cible affiche un message proposant d'accéder à des informations relatives au lieu où elle se trouve ou bien au service proposé. La personne compose (ou envoie) alors cet identifiant (de communication) avec son téléphone mobile MS et des informations générales s'affichent sur l'écran cible EC. Par exemple, l'une des fenêtres d'informations affichées propose à la personne de sélectionner une langue. Si cette personne est étrangère, elle va sélectionner avec son téléphone mobile MS la langue de son choix dans une liste proposée. Les informations vont alors s'afficher sur l'écran cible EC dans la langue sélectionnée, et la personne peut éventuellement accéder à d'autres informations (éventuellement plus détaillées) en composant (ou envoyant) un autre identifiant (de communication) affiché. On notera que des images vidéo peuvent être affichées sur l'écran cible EC, et qu'elles peuvent être éventuellement accompagnées d'une bande son, dans la langue sélectionnée, qui peut être transmise au téléphone mobile MS afin qu'il la diffuse sur son haut-parleur évitant ainsi de déranger les autres personnes situées à proximité de l'écran cible EC.

Le serveur SR est de préférence révisé sous la forme d'une combinaison de circuits électroniques (« hardware ») et de modules logiciels (« software »). Mais, il pourrait être réalisé sous la forme de circuits électroniques, voire même essentiellement de modules logiciels s'il est intégré dans un équipement (comme par exemple un équipement réseau ou un terminal de communication fixe).

L'invention offre un certain nombre d'avantages, parmi lesquels:
- un nouveau type de service basé sur la localisation (LBS) pour des terminaux de communication radio de n'importe quelle génération, qui ne nécessite pas d'adaptation ou d'interface et qui ne nécessite pas de localisation,
- elle donne accès à un grand nombre d'applications,
- elle ne nécessite aucune connaissance ou compétence particulière de la part des personnes qui l'utilisent,
- une personnalisation des informations, notamment de la langue d'affichage et/ou de diffusion,
- le mélange sur un téléviseur (ou écran) numérique des contenus qui sont diffusés (éventuellement au moyen d'un service de vidéo à la demande (ou VoD)), par exemple de type programmes de télévision, avec des informations et/ou applications (éventuellement des jeux) requises par l'usager et transmises par exemple par l'Internet.

L'invention ne se limite pas aux modes de réalisation de serveur et de procédé d'ouverture de session de communication décrits ci-avant, seulement à titre d'exemple, mais elle englobe toutes les variantes que pourra envisager l'homme de l'art dans le cadre des revendications ci-après.

Ainsi, l'invention s'applique également à la fourniture d'informations relatives à des publicités affichées sur des écrans cibles, ainsi qu'au déclenchement d'applications du type de celles présentées ci-avant.

## Revendications

1. Procédé d'ouverture d'une session de communication entre un terminal de communication radio (MS), connecté un réseau de communication radio (R1), et un terminal de communication fixe (TC), couplé à au moins un écran cible (EC) et connecté à un réseau de communication (R2), **caractérisé en ce qu'**il consiste à envoyer avec ledit terminal de communication radio (MS) un identifiant affiché sur ledit écran cible (EC) pour ouvrir une session de communication entre ledit terminal de communication radio (MS) et ledit terminal de communication fixe (TC), puis à contrôler l'affichage d'informations sur ledit écran cible (EC) au moyen dudit terminal de communication radio (MS).

2. Procédé selon la revendication 1, **caractérisé en ce que** l'on transfère le contrôle de l'affichage d'informations audit terminal de communication radio (MS).

3. Procédé selon la revendication 2, **caractérisé en ce que** l'on envoie avec ledit terminal de communication radio (MS) un identifiant qui est choisi dans une liste affichée sur ledit écran cible (EC) pour joindre un serveur (SR), puis on ouvre ladite session de communication au moyen dudit sen/eur (SR), et on procède au transfert du contrôle d'affichage au moyen dudit serveur (SR).

4. Procédé selon l'une des revendications 1 à 3, **caractérisé en ce qu'**en présence d'un identifiant de type identifiant de communication on compose ledit identifiant avec ledit terminal de communication radio (MS).

5. Procédé selon l'une des revendications 1 à 4, **caractérisé en ce que** l'on utilise des moyens (MI) dudit terminal de communication radio (MS), constituant une interface homme/machine, pour effectuer des opérations permettant d'agir sur certaines au moins des informations affichées sur ledit écran cible (EC) et/ou de provoquer l'affichage sur ledit écran cible (EC) de certains informations stockées.

6. Procédé selon l'une des revendications 1 à 5, **caractérisé en ce qu'**en cas d'affichage sur ledit écran cible (EC) d'informations accompagnées d'un autre identifiant donnant accès à d'autres informations stockées, on provoque l'affichage de ces autres informations stockées en envoyant cet autre identifiant avec ledit terminal de communication radio (MS).

7. Procédé selon l'une des revendications 1 à 6, **caractérisé en ce que** l'on affiche sur un écran d'affichage (EA) dudit terminal de communication radio (MS) certaines au moins des informations qui sont affichées sur ledit écran cible (EC).

8. Procédé selon l'une des revendications 1 à 7, **caractérisé en ce que** l'on déclenche au moine une application de télécommunications choisie dans un groupe comprenant au moins une application de mise en communication de l'usager dudit terminal de communication radio (MS) avec un autre usager ou un équipement de communication, et une application permettant de stocker certaines au moins des informations qui sont affichées sur ledit écran cible (EC) dans des moyens de stockage (MM2) désignés par l'usager dudit terminal de communication radio (MS).

9. Procédé selon l'une des revendications 1 à 8, **caractérisé en ce que** l'on transmet audit terminal de communication radio (MS) des messages audio associés aux informations affichées afin qu'ils puissent être écoutés par son usager.

10. Procédé selon l'une des revendications 1 à 9, **caractérisé en ce que** l'on met fin à ladite session de communication en cas de détection d'une absence d'opération de contrôle d'affichage avec ledit terminal de communication radio (MS) pendant une durée supérieure à un seuil choisi.

11. Procédé selon l'une des revendications 1 à 10, **caractérisé en ce que** l'on met fin à ladite session de communication lorsque l'usager dudit terminal de communication radio (MS) interrompt la communication.

12. Procédé selon l'une des revendications 1 à 11, **caractérisé en ce que** l'on facture à l'usager dudit terminal de communication radio (MS) ladite prise de contrôle de l'affichage.

13. Procédé selon l'une des revendications 1 à 12, **caractérisé en ce que** l'on stocke dans ledit terminal de communication fixe (TC) certaines au moins desdites informations destinées à être affichées sur ledit écran cible (EC).

14. Procédé selon l'une des revendications 3 à 13, **caractérisé en ce que** l'on stocke dans ledit serveur (SR) certaines au moins desdites informations destinées à être affichées sur ledit écran cible (EC).

15. Serveur de réseau de communication (SG), **caractérisé en ce qu'**il est agencé, en cas d'établissement d'une communication avec un terminal de communication radio (MS) connecté à un réseau de communication radio (R1) auquel il est couplé, consécutivement à renvoi par ledit terminal de communication radio (MS) d'un identifiant affiché sur un écran cible (EC) couplé à un terminal de communication fixe (TC) connecté à un réseau de communication (R2), pour ouvrir une session de communication entre ledit terminal de communication radio (MS) et ledit terminal de communication fixe (TC), puis pour transférer audit terminal de communication radio (MS) le contrôle de l'affichage d'informations sur ledit écran cible (EC).

16. Serveur selon la revendication 15, **caractérisé en ce qu'**il est agencé, en cas d'envoi par ledit terminal de communication radio (MS) pendant ladite session de communication d'un autre identifiant affiché sur ledit écran cible (EC) et donnant accès à d'autres informations stockées, pour autoriser l'accès à ces autres informations stockées de sorte qu'elles puissent être affichées sur ledit écran cible (EC).

17. Serveur selon l'une des revendications 15 et 16, **caractérisé en ce qu'**il est agencé pour autoriser la transmission audit terminal de communication radio (MS) de certaines au moins des informations qui sont affichées sur ledit écran cible (EC), en vue de leur affichage sur un écran d'affichage (EA) dudit terminal de communication radio (MS).

18. Serveur selon l'une des revendications 15 à 17, **caractérisé en ce qu'**il est agencé pour déclencher au moins une application de télécommunications choisie dans un groupe comprenant au moins une application de mise en communication de l'usager dudit terminal de communication radio (MS) avec un autre usager ou un équipement de communication, et une application permettant de stocker certaines au moins des informations qui sont affichées sur ledit écran cible (EC) dans des moyens de stockage (MM2) désignés par l'usager dudit terminal de communication radio (MS).

19. Serveur selon l'une des revendications 15 à 18, **caractérisé en ce qu'**il est agencé pour transmettre audit terminal de communication radio (MS) des messages audio associés aux informations affichées sur ledit écran cible (EC) afin qu'ils puissent être écoutés par son usager.

20. Serveur selon l'une des revendications 15 à 19, **caractérisé en ce qu'**il est agencé pour mettre fin à ladite session de communication en cas de détection d'une absence d'opération de contrôle d'affichage avec ledit terminal de communication radio (MS) pendant une durée supérieure à un seuil choisi.

21. Serveur selon l'une des revendications 15 à 20, **caractérisé en ce qu'**il est agencé pour mettre fin à ladite session de communication lorsque l'usager dudit terminal de communication radio (MS) interrompt la communication.

22. Serveur selon l'une des revendications 15 à 21, **caractérisé en ce qu'**il est agencé pour contrôler la facturation à l'usager dudit terminal de communication radio (MS) de la prise de contrôle de l'affichage,

23. Serveur selon l'une des revendications 15 à 22, **caractérisé en ce qu'**il comprend des moyens de stockage (MM1) stockant certaines au moins desdites informations destinées à être affichées sur ledit écran cible (EC).

24. Serveur selon l'une des revendications 15 à 23, **caractérisé en ce qu'**il est agencé pour autoriser la transmission à une adresse désignée par l'usager dudit terminal de communication radio (MS) de certaines au moins des informations qui sont affichées sur ledit écran cible (EC), en vue de leur stockage dans des moyens de stockage (MM2).
